# EUROPEAN PATENT SPECIFICATION

(11) **EP 2 384 200 B1**
(45) Date of publication and mention of the grant of the patent: **10.07.2013**
(21) Application number: 10702554.6
(22) Date of filing: 01.02.2010
(51) Int. Cl.: A61K 38/45, C12N 9/10, A61P 13/04, A61P 13/12, A61P 13/00

(54) **ALANINE-GLYOXYLATE AMINOTRANSFERASE THERAPEUTICS**
ALANINGLYOXYLAT-AMINOTRANSFERASE-THERAPEUTIK
TRAITEMENTS À BASE D'ALANINE-GLYOXYLATE AMINOTRANSFÉRASE

(30) Priority: 30.01.2009 EP 09151795
(43) Date of publication of application: 09.11.2011
(73) Proprietor: UniQure IP B.V., 1105 BA Amsterdam (NL)
(72) Inventor: RODRIGUEZ PENA, Maria Sol, 1052 Le Mont-sur-Lausanne (CH); PETRY, Harald, NL-1105 BA Amsterdam (NL); TWISK, Jaap, NL-1105 BA Amsterdam (NL); VAN DEVENTER, Sander Jan Hendrik, 1105 BA Amsterdam (NL); SALIDO RUIZ, Eduardo Carlos, ES-38320 Tenerife (ES); TORRES RAMIREZ, Armando, ES-38320 Tenerife (ES)
(74) Representative: Nederlandsch Octrooibureau
(86) International application number: PCT/NL2010/050044
(87) International publication number: WO 2010/087709

(56) References cited:
- TOUSSAINT C ET AL: "Primary hyperoxalurias" NEPHROLOGIE, EDITIONS MEDECINE ET HYGIENE, GENEVA, CH, vol. 16, no. 6, 1 January 1995 (1995-01-01), pages 399-406, XP009118547 ISSN: 0250-4960
- COULTER-MACKIE M B ET AL: "Overexpression of human alanine:glyoxylate aminotransferase in Escherichia coli: renaturation from guanidine-HCl and affinity for pyridoxal phosphate co-factor" PROTEIN EXPRESSION AND PURIFICATION, ACADEMIC PRESS, SAN DIEGO, CA, vol. 41, no. 1, 1 May 2005 (2005-05-01), pages 18-26, XP004817037 ISSN: 1046-5928
- WATTS ET AL: "Alanine glyoxylate aminotransferase deficiency: Biochemical and molecular genetic lessons from the study of a human disease" ADVANCES IN ENZYME REGULATION, PERGAMON PRESS, OXFORD, GB, vol. 32, 1 January 1992 (1992-01-01), pages 309-327, XP022660237 ISSN: 0065-2571 [retrieved on 1992-01-01]
- PURDUE, P. EDWARD ET AL: "Identification of mutations associated with peroxisome-to-mitochondrion mistargeting of alanine/glyoxylate aminotransferase in primary hyperoxaluria type 1" JOURNAL OF CELL BIOLOGY , 111(6, PT. 1), 2341-51 CODEN: JCLBA3; ISSN: 0021-9525, 1990, XP002533385

### SEQUENCE LISTING

<110> Amsterdam Molecular Therapeutics (AMT) IP B.V.
<120> Alanine-glyoxylate aminotransferase therapeutics
<130> P6022296PCT
<150> EP09151795.3
   <151> 2009-01-30
<160> 8
<170> PatentIn version 3.3
<210> 1
   <211> 1179
   <212> DNA
   <213> Homo sapiens
<220>
   <221> misc_feature
   <223> human AGXT I340M allele (rs4426527)
<220>
   <221> CDS
   <222> (1)..(1179)
<400> 1
   atg gcc tct cac aag ctg ctg gtg acc ccc ccc aag gcc ctg ctc aag     48
   Met Ala Ser His Lys Leu Leu Val Thr Pro Pro Lys Ala Leu Leu Lys
   1               5                   10                  15
   ccc ctc tcc atc ccc aac cag ctc ctg ctg ggg cct ggt cct tcc aac     96
   Pro Leu Ser Ile Pro Asn Gln Leu Leu Leu Gly Pro Gly Pro Ser Asn
               20                  25                  30
   ctg cct cct cgc atc atg gca gcc ggg ggg ctg cag atg atc ggg tcc    144
   Leu Pro Pro Arg Ile Met Ala Ala Gly Gly Leu Gln Met Ile Gly Ser
           35                  40                  45
   atg agc aag gat atg tac cag atc atg gac gag atc aag gaa ggc atc    192
   Met Ser Lys Asp Met Tyr Gln Ile Met Asp Glu Ile Lys Glu Gly Ile
       50                  55                  60
   cag tac gtg ttc cag acc agg aac cca ctc aca ctg gtc atc tcc ggc    240
   Gln Tyr Val Phe Gln Thr Arg Asn Pro Leu Thr Leu Val Ile Ser Gly
   65                  70                  75                  80
   tcg gga cac tgt gcc ctg gag gcc gcc ctg gtc aat gtg ctg gag cct    288
   Ser Gly His Cys Ala Leu Glu Ala Ala Leu Val Asn Val Leu Glu Pro
                   85                  90                  95
   ggg gac tcc ttc ctg gtt ggg gcc aat ggc att tgg ggg cag cga gcc    336
   Gly Asp Ser Phe Leu Val Gly Ala Asn Gly Ile Trp Gly Gln Arg Ala
               100                 105                 110
   gtg gac atc ggg gag cgc ata gga gcc cga gtg cac ccg atg acc aag    384
   Val Asp Ile Gly Glu Arg Ile Gly Ala Arg Val His Pro Met Thr Lys
           115                 120                 125
   gac ccc gga ggc cac tac aca ctg cag gag gtg gag gag ggc ctg gcc    432
   Asp Pro Gly Gly His Tyr Thr Leu Gln Glu Val Glu Glu Gly Leu Ala
       130                 135                 140
   cag cac aag cca gtg ctg ctg ttc tta acc cac ggg gag tcg tcc acc    480
   Gln His Lys Pro Val Leu Leu Phe Leu Thr His Gly Glu Ser Ser Thr
   145                 150                 155                 160
   ggc gtg ctg cag ccc ctt gat ggc ttc ggg gaa ctc tgc cac agg tac      528
   Gly Val Leu Gln Pro Leu Asp Gly Phe Gly Glu Leu Cys His Arg Tyr
                   165                 170                 175
   aag tgc ctg ctc ctg gtg gat tcg gtg gca tcc ctg ggc ggg acc ccc      576
   Lys Cys Leu Leu Leu Val Asp Ser Val Ala Ser Leu Gly Gly Thr Pro
               180                 185                 190
   ctt tac atg gac cgg caa ggc atc gac atc ctg tac tcg ggc tcc cag      624
   Leu Tyr Met Asp Arg Gln Gly Ile Asp Ile Leu Tyr Ser Gly Ser Gln
           195                 200                 205
   aag gcc ctg aac gcc cct cca ggg acc tcg ctc atc tcc ttc agt gac      672
   Lys Ala Leu Asn Ala Pro Pro Gly Thr Ser Leu Ile Ser Phe Ser Asp
       210                 215                 220
   aag gcc aaa aag aag atg tac tcc cgc aag acg aag ccc ttc tcc ttc      720
   Lys Ala Lys Lys Lys Met Tyr Ser Arg Lys Thr Lys Pro Phe Ser Phe
   225                 230                 235                 240
   tac ctg gac atc aag tgg ctg gcc aac ttc tgg ggc tgt gac gac cag      768
   Tyr Leu Asp Ile Lys Trp Leu Ala Asn Phe Trp Gly Cys Asp Asp Gln
                   245                 250                 255
   ccc agg atg tac cat cac aca atc ccc gtc atc agc ctg tac agc ctg      816
   Pro Arg Met Tyr His His Thr Ile Pro Val Ile Ser Leu Tyr Ser Leu
               260                 265                 270
   aga gag agc ctg gcc ctc att gcg gaa cag ggc ctg gag aac agc tgg      864
   Arg Glu Ser Leu Ala Leu Ile Ala Glu Gln Gly Leu Glu Asn Ser Trp
           275                 280                 285
   cgc cag cac cgc gag gcc gcg gcg tat ctg cat ggg cgc ctg cag gca      912
   Arg Gln His Arg Glu Ala Ala Ala Tyr Leu His Gly Arg Leu Gln Ala
       290                 295                 300
   ctg ggg ctg cag ctc ttc gtg aag gac ccg gcg ctc cgg ctt ccc aca      960
   Leu Gly Leu Gln Leu Phe Val Lys Asp Pro Ala Leu Arg Leu Pro Thr
   305                 310                 315                 320
   gtc acc act gtg gct gta ccc gct ggc tat gac tgg aga gac atc gtc     1008
   Val Thr Thr Val Ala Val Pro Ala Gly Tyr Asp Trp Arg Asp Ile Val
                   325                 330                 335
   agc tac gtc atg gac cac ttc gac att gag atc atg ggt ggc ctt ggg     1056
   Ser Tyr Val Met Asp His Phe Asp Ile Glu Ile Met Gly Gly Leu Gly
               340                 345                 350
   ccc tcc acg ggg aag gtg ctg cgg atc ggc ctg ctg ggc tgc aat gcc     1104
   Pro Ser Thr Gly Lys Val Leu Arg Ile Gly Leu Leu Gly Cys Asn Ala
           355                 360                 365
   acc cgc gag aat gtg gac cgc gtg acg gag gcc ctg agg gcg gcc ctg     1152
   Thr Arg Glu Asn Val Asp Arg Val Thr Glu Ala Leu Arg Ala Ala Leu
       370                 375                 380
   cag cac tgc ccc aag aag aag ctg tga                                 1179
   Gln His Cys Pro Lys Lys Lys Leu
   385                 390
<210> 2
   <211> 392
   <212> PRT
   <213> Homo sapiens
<400> 2
   Met Ala Ser His Lys Leu Leu Val Thr Pro Pro Lys Ala Leu Leu Lys
   1               5                   10                  15
   Pro Leu Ser Ile Pro Asn Gln Leu Leu Leu Gly Pro Gly Pro Ser Asn
               20                  25                  30
   Leu Pro Pro Arg Ile Met Ala Ala Gly Gly Leu Gln Met Ile Gly Ser
           35                  40                  45
   Met Ser Lys Asp Met Tyr Gln Ile Met Asp Glu Ile Lys Glu Gly Ile
       50                  55                  60
   Gln Tyr Val Phe Gln Thr Arg Asn Pro Leu Thr Leu Val Ile Ser Gly
   65                  70                  75                  80
   Ser Gly His Cys Ala Leu Glu Ala Ala Leu Val Asn Val Leu Glu Pro
                   85                  90                  95
   Gly Asp Ser Phe Leu Val Gly Ala Asn Gly Ile Trp Gly Gln Arg Ala
               100                 105                 110
   Val Asp Ile Gly Glu Arg Ile Gly Ala Arg Val His Pro Met Thr Lys
           115                 120                 125
   Asp Pro Gly Gly His Tyr Thr Leu Gln Glu Val Glu Glu Gly Leu Ala
       130                 135                 140
   Gln His Lys Pro Val Leu Leu Phe Leu Thr His Gly Glu Ser Ser Thr
   145                 150                 155                 160
   Gly Val Leu Gln Pro Leu Asp Gly Phe Gly Glu Leu Cys His Arg Tyr
                   165                 170                 175
   Lys Cys Leu Leu Leu Val Asp Ser Val Ala Ser Leu Gly Gly Thr Pro
               180                 185                 190
   Leu Tyr Met Asp Arg Gln Gly Ile Asp Ile Leu Tyr Ser Gly Ser Gln
           195                 200                 205
   Lys Ala Leu Asn Ala Pro Pro Gly Thr Ser Leu Ile Ser Phe Ser Asp
       210                 215                 220
   Lys Ala Lys Lys Lys Met Tyr Ser Arg Lys Thr Lys Pro Phe Ser Phe
   225                 230                 235                 240
   Tyr Leu Asp Ile Lys Trp Leu Ala Asn Phe Trp Gly Cys Asp Asp Gln
                   245                 250                 255
   Pro Arg Met Tyr His His Thr Ile Pro Val Ile Ser Leu Tyr Ser Leu
               260                 265                 270
   Arg Glu Ser Leu Ala Leu Ile Ala Glu Gln Gly Leu Glu Asn Ser Trp
                   275                     280                     285
   Arg Gln His Arg Glu Ala Ala Ala Tyr Leu His Gly Arg Leu Gln Ala
       290                     295                     300
   Leu Gly Leu Gln Leu Phe Val Lys Asp Pro Ala Leu Arg Leu Pro Thr
   305                     310                     315                     320
   Val Thr Thr Val Ala Val Pro Ala Gly Tyr Asp Trp Arg Asp Ile Val
                   325                     330                     335
   Ser Tyr Val Met Asp His Phe Asp Ile Glu Ile Met Gly Gly Leu Gly
               340                     345                     350
   Pro Ser Thr Gly Lys Val Leu Arg Ile Gly Leu Leu Gly Cys Asn Ala
           355                     360                     365
   Thr Arg Glu Asn Val Asp Arg Val Thr Glu Ala Leu Arg Ala Ala Leu
       370                     375                     380
   Gln His Cys Pro Lys Lys Lys Leu
   385                 390
<210> 3
   <211> 673
   <212> DNA
   <213> Artificial
<220>
   <223> Human AAT promoter combined with the mouse albumin gene enhancer (Ealb) element
<400> 3
   tcgaggttcc tagattacac tacacattct gcaagcatag cacagagcaa tgttctactt      60
   taattacttt cattttcttg tatcctcaca gcctagaaaa taacctgcgt tacagcatcc     120
   actcagtatc ccttgagcat gaggtgacac tacttaacat agggacgaga tggtactttg     180
   tgtctcctgc tctgtcagca gggcacagta cttgctgata ccagggaatg tttgttctta     240
   aataccatca ttccggacgt gtttgccttg gccagttttc catgtacatg cagaaagaag     300
   tttggactga tcaatacagt cctctgcctt taaagcaata ggaaaaggcc aacttgtcta     360
   cgtttagtat gtggctgtag atctgtaccc gccaccccct ccaccttgga cacaggacgc     420
   tgtggtttct gagccaggta caatgactcc tttcggtaag tgcagtggaa gctgtacact     480
   gcccaggcaa agcgtccggg cagcgtaggc gggcgactca gatcccagcc agtggactta     540
   gcccctgttt gctcctccga taactggggt gaccttggtt aatattcacc agcagcctcc     600
   cccgttgccc ctctggatcc actgcttaaa tacggacgag gacagggccc tgtctcctca     660
   gcttcaggca cca                                                        673
<210> 4
   <211> 96
   <212> DNA
   <213> Homo sapiens
<400> 4
   tccgtcgacc cgggcggccg atcgctagcc tttgaatgta accaatccta ctaataaacc 60
   agttctgaag gtgttgtgtg tgcgcgtgtg gagttg 96
<210> 5
   <211> 66
   <212> DNA
   <213> Artificial
<220>
   <223> Synthetic polyA Insulator
<400> 5
   aattcaataa agagctctta ttttcattct cgaggtgtgg ttggttttct tgtgtggggg 60
   cggatc 66
<210> 6
   <211> 10
   <212> RNA
   <213> Artificial
<220>
   <223> Kozak consensus sequence
<400> 6
   gccrccaugg 10
<210> 7
   <211> 145
   <212> DNA
   <213> adeno-associated virus 2
<400> 7
   ttggccactc cctctctgcg cgctcgctcg ctcactgagg ccgggcgacc aaaggtcgcc 60
   cgacgcccgg gctttgcccg ggcggcctca gtgagcgagc gagcgcgcag agagggagtg 120
   gccaactcca tcactagggg ttcct 145
<210> 8
   <211> 146
   <212> DNA
   <213> adeno-associated virus 2
<400> 8
   ttggccactc cctctctgcg cgctcgctcg ctcactgagg ccgcccgggc aaagcccggg     60
   cgtcgggcga cctttggtcg cccggcctca gtgagcgagc gagcgcgcag agagggagtg    120
   gccaactcca tcactagggg ttcccc                                         146

## Description

### Field of the invention

The present invention is in the fields of medicine and molecular biology. More specifically the invention relates to novel protein and nucleic acid therapeutics based on alanine-glyoxylate aminotransferase (AGXT), in particular a variant of AGXT, including therapeutics delivered by gene therapy, and methods for use of these therapeutics in treating and preventing conditions caused by absence or deficiency of alanine-glyoxylate aminotransferase, including primary hyperoxaluria type I.

### Background of the invention

Primary hyperoxaluria type I (PH1) (OMIM #259900) is a rare metabolic disorder, inherited in an autosomal recessive manner. PHI is characterized by a deficiency of the hepatic enzyme alanine-glyoxylate aminotransferase (AGXT or AGT; EC 2.6.1.44), that is usually caused by a mutation in the gene encoding on 2q36-q37 (Genbank Acc. No. NM_000030, version 14 June 2008). An AGXT deficiency results in failure to detoxify glyoxylate, with overproduction of oxalate. AGXT converts glyoxylate to glycine, using alanine as the donor of the amino group, and the cofactor pyridoxal-phosphate. High levels of oxalate in PHI patients are excreted by the kidneys, which undergo progressive deterioration as a result of calcium oxalate deposition. After kidney failure, oxalate levels raise to the point of systemic oxalosis, a life threatening condition. Currently, the most effective treatment for PH1 is pre-emptive liver transplantation or combined liver and kidney transplantation. However, this treatment has its own limitations including the scarce supply of suitable organs, significant morbidity and mortality, and chronic exposure to immunosuppressive drugs.

Thus, new treatments for PHI are needed. It is an object of the present invention to provide to novel protein and nucleic acid therapeutics based on variants of alanine-glyoxylate aminotransferase (AGXT) and methods for use of these therapeutics in treating and preventing conditions caused by absence or deficiency of alanine-glyoxylate aminotransferase, including primary hyperoxaluria type I.

### Summary of the invention

The invention relates to an AGXT I340M therapeutic, for use as a medicament or for use in a method of treatment. Such a therapeutic may be used in the treatment of an AGXT-responsive condition, whereby, the AGXT-responsive condition is primary hyperoxaluria type I.

The AGXT I340M therapeutic is selected from the group consisting of: a) an AGXT I340M protein comprising an amino acid sequence having at least 90% sequence identity to SEQ ID NO: 2 when optimally aligned over their entire length using the GAP program, and wherein the AGXT I340M protein comprises a methionine at a position corresponding to position 340 in SEQ ID NO: 2; b) a nucleic acid molecule comprising an nucleotide sequence encoding an AGXT I340M protein as defined in a); and, c) a virion of a viral gene therapy vector comprising a nucleic acid molecule as defined in b).

An AGXT therapeutic based on the wild type (not claimed) may be defined in a similar fashion as to the AGXT I340M therapeutic described above, except that such a therapeutic would comprise isoleucine (or an amino acid other than methionine) at a position corresponding to position 340 in SEQ ID NO: 2 (i.e. may be based on the AGXT wild type sequence). Herein, that which is described in relation to an AGXT I340M therapeutic may be applied similarly to an AGXT therapeutic based on the wild type AGXT sequence.

An AGXT I340M therapeutic according to the invention may further comprise or encode amino acid substitutions selected from the group consisting of: a) a leucine at a position corresponding to position 11 in SEQ ID NO: 2; b) an arginine at a position corresponding to position 41 in SEQ ID NO: 2; c) an isoleucine at a position corresponding to position 152 in SEQ ID NO: 2; d) an arginine at a position corresponding to position 170 in SEQ ID NO: 2; e) a threonine at a position corresponding to position 244 in SEQ ID NO: 2; f) a threonine at a position corresponding to position 294 in SEQ ID NO: 2; g) an isoleucine at a position corresponding to position 326 in SEQ ID NO: 2; h) combinations of one or more of the substitutions amino acid b) to g); and, i) the combination of the substitutions a) and f).

The invention also relates to a nucleic acid construct comprising a nucleotide sequence coding for the AGXT I340M protein of the invention, wherein the nucleotide sequence is operably linked to a promoter for expression in human cells and wherein, optionally, the promoter is not a promoter from a human AGXT gene. Preferably, the promoter is a liver-specific promoter. The liver-specific promoter may be selected from the group consisting of an α1-anti-trypsin (AAT) promoter, a thyroid hormone-binding globulin promoter, an albumin promoter, a thyroxin-binding globulin (TBG) promoter, an Hepatic Control Region (HCR)-ApoCII hybrid promoter, an HCR-hAAT hybrid promoter, an AAT promoter combined with the mouse albumin gene enhancer (Ealb) element, an apolipoprotein E promoter and a promoter that has the sequence of SEQ ID NO: 3. The nucleic acid construct according to this aspect preferably is a viral gene therapy vector, more preferably a parvoviral vector.

The invention further pertains to a parvoviral virion comprising a nucleic acid construct encoding the AGXT I340M therapeutic.

Also, the invention relates to a pharmaceutical composition comprising an AGXT therapeutic, nucleic acid construct or parvoviral virion of the invention and a pharmaceutically acceptable carrier.

The invention also provides a method for treating an AGXT-responsive condition wherein the method comprises the step of administering an effective amount of an AGXT I340M therapeutic, a nucleic acid construct, a parvoviral virion or a pharmaceutical composition of the invention to a subject having a said AGXT-responsive condition, for example, primary hyperoxaluria type I.

In addition, the invention provides, use of an AGXT I340M therapeutic, a nucleic acid construct, a parvoviral virion or a pharmaceutical composition of the invention in the manufacture of a medicament for use in the treatment of an AGXT-responsive condition, for example, primary hyperoxaluria type I.

### Description of the figures

Fig. 1 shows a schematic of the rAAV vectors used. rAAV vectors were constructed by inserting the human *AGXT* cDNA into a pro-AAV2 vector plasmid, under the control of a hybrid EalbAAT liver-specific promoter. Downstream of the AGXT cDNA (or GFP) a bGH polyadenylation signal was included. Some constructs included a WPRE post-transcriptional regulatory element. The expression cassette was flanked by inverted terminal repeats from AAV2
Fig. 2 shows urinary excretion of Oxalate in male AGXT^{-/-} mice treated with ethylene glycol (EG) after the administration of 5x10¹² gc/kg of therapeutic vector AA V8.Ealb-hAAT-AGXT-WPRE or control vector AAV8.Ealb-hAAT-GFP-WPRE.
Fig. 3a shows Western blot analysis of AGXT expression within livers of AGXT-/- mice (of both genders) 8 weeks after intravenous injection of 5x10e12 vector genomes per kg AAV8 or AAV5 vector (the latter also administered at the ten-fold lower dose 5x10e11vg/kg).
Figs. 3b and 3c (the insert) show expression of AGT in liver samples from male AGXT^{-/-} mice injected with the AAV8.Ealb-hAAT-AGXT-WPRE or control vector AAV8.Ealb-hAAT-GFP-WPRE (100x magnification) respectively.
Fig. 3d shows results obtained by immunohistochemical staining of frozen liver sections, with percentages of hepatocytes transduced around 90% for AAV8 and the highest dose of AAV5, 60% for 5x10¹² AAV5 particles/kg, and down to 10% (and less than 1% in females) for 5x10¹¹ AAV5 particles/kg.

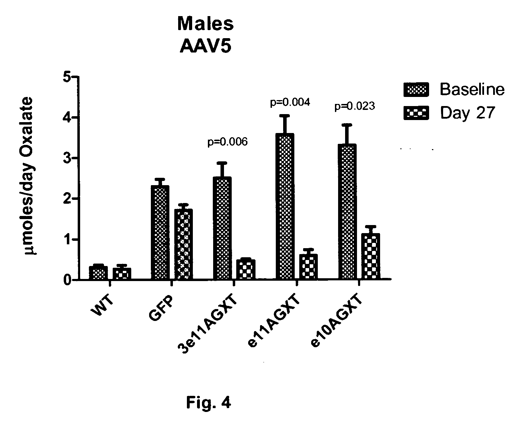

Fig. 4 shows urinary excretion of Oxalate in male AGXT^{-/-} mice treated with ethylene glycol (EG) after the administration of either 1x10¹⁰, 1x10¹¹ and 3x10¹¹ gc/kg of therapeutic vector AAV5.Ealb-hAAT-AGXT-WPRE or control vector AAV5.Ealb-hAAT-GFP-WPRE (at the day of injection and 27 days later). A marked reduction in oxaluria is observed in mice treated with all three doses tested, although higher doses showed the most statistically significant reduction, achieving oxalate excretion levels similar to wild type mice. As expected, AgxtKO mice injected with GFP-AAV5 control viral particles did not show significant oxaluria reduction.

### Description of the invention

### Definitions

A "nucleic acid construct" is herein understood to mean a man-made nucleic acid molecule resulting from the use of recombinant DNA technology. A nucleic acid construct is a nucleic acid molecule, either single- or double-stranded, which has been modified to contain segments of nucleic acids, which are combined and juxtaposed in a manner, which would not otherwise exist in nature. A nucleic acid construct usually is a "vector", i.e. a nucleic acid molecule which may be used to deliver exogenously created nucleic acids, such as DNA into a host cell.

One type of nucleic acid construct is an "expression cassette" or "expression vector". These terms refers to nucleotide sequences that are capable of effecting expression of a gene in a host cell or a host organism compatible with such sequences. Expression cassettes or expression vectors typically include at least suitable transcription regulatory sequences and optionally, 3' transcription termination signals. Additional factors necessary or helpful in effecting expression may also be present, such as expression enhancer elements.

The term "homologous" when used to indicate the relation between a given (recombinant) nucleic acid or polypeptide molecule and a given host organism or host cell, is understood to mean that in nature the nucleic acid or polypeptide molecule is produced by a host cell or an organism of the same species. The term "heterologous" may be used to indicate that in nature the nucleic acid or polypeptide molecule is produced by a host cell or an organism of a different species.

As used herein, the term "operably linked" refers to a linkage of polynucleotide (or polypeptide) elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame.

"Expression control sequence" refers to a nucleic acid sequence that regulates the expression of a nucleotide sequence to which it is operably linked. An expression control sequence is "operably linked" to a nucleotide sequence when the expression control sequence controls and regulates the transcription and/or the translation of the nucleotide sequence. Thus, an expression control sequence can include promoters, enhancers, internal ribosome entry sites (IRES), transcription terminators, a start codon in front of a protein-encoding gene, splicing signal for introns, and stop codons. The term "expression control sequence" is intended to include, at a minimum, a sequence whose presence is designed to influence expression, and can also include additional advantageous components. For example, leader sequences and fusion partner sequences are expression control sequences. The term can also include the design of the nucleic acid sequence such that undesirable, potential initiation codons in and out of frame, are removed from the sequence. It can also include the design of the nucleic acid sequence such that undesirable potential splice sites are removed. It includes sequences or polyadenylation sequences (pA) which direct the addition of a polyA tail, i.e., a string of adenine residues at the 3'-end of a mRNA, sequences referred to as polyA sequences. It also can be designed to enhance mRNA stability. Expression control sequences which affect the transcription and translation stability, e.g., promoters, as well as sequences which effect the translation, e.g., Kozak sequences, are known in insect cells. Expression control sequences can be of such nature as to modulate the nucleotide sequence to which it is operably linked such that lower expression levels or higher expression levels are achieved.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter, including e.g. attenuators or enhancers, but also silencers.

A "constitutive" promoter is a promoter that is active in most tissues under most physiological and developmental conditions. An "inducible" promoter is a promoter that is physiologically or developmentally regulated, e.g. by the application of a chemical inducer. A "tissue specific" promoter is only active, substantially only active or predominantly active in specific types of tissues or cells.

A "3' UTR" or "3' non-translated sequence" (also often referred to as 3' untranslated region, or 3'end) refers to the nucleic acid sequence found downstream of the coding sequence of a gene, which comprises, for example, a transcription termination site and (in most, but not all eukaryotic mRNAs) a polyadenylation signal (such as e.g. AAUAAA or variants thereof). After termination of transcription, the mRNA transcript may be cleaved downstream of the polyadenylation signal and a poly(A) tail may be added, which is involved in the transport of the mRNA to the cytoplasm (where translation takes place).

The terms "substantially identical", "substantial identity" or "essentially similar" or "essential similarity" means that two peptide or two nucleotide sequences, when optimally aligned, such as by the programs GAP or BESTFIT using default parameters, share at least a certain percentage of sequence identity as defined elsewhere herein. GAP uses the Needleman and Wunsch global alignment algorithm to align two sequences over their entire length, maximizing the number of matches and minimizes the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). It is clear than when RNA sequences are said to be essentially similar or have a certain degree of sequence identity with DNA sequences, thymine (T) in the DNA sequence is considered equal to uracil (U) in the RNA sequence. Sequence alignments and scores for percentage sequence identity may be determined using computer programs, such as the GCG Wisconsin Package, Version 10.3, available from Accelrys Inc., 9685 Scranton Road, San Diego, CA 92121-3752 USA or the open-source software Emboss for Windows (current version 2.7.1-07). Alternatively percent similarity or identity may be determined by searching against databases such as FASTA, BLAST, etc.

### Detailed description of the invention

AGXT 1340M (a variant rarely seen in the general population) results in a significantly higher activity than the most common major allele. Herein, it is shown that a single tail vein administration of an AAV gene therapy vector incorporating nucleic acids encoding AGXT with I340M result in sustained correction of the primary hyperoxaluria type I (PH1) phenotype in a mouse model, without evidence of liver damage or toxicity.

This AAV-based gene transfer approach resulted in liver expression of the human AGXT I340M sufficiently robust so as to overcome the potential problem posed by the continued oxalate production of non-transduced hepatocytes. Further, the expressed gene is correctly targeted to the peroxisome in the mouse hepatocyte, and it complements the deficit of the mouse Agxt1KO model.

Accordingly, the invention pertains to an AGXT I340M therapeutic. That is to say, the invention involves the precognition of important advantages that may be obtained through therapeutic treatments comprising the administration of therapeutics derived from the AGXT I340M protein, and nucleic acid sequences encoding the AGXT I340M protein.

In the context of this invention an AGXT I340M therapeutic, may include AGXT I340M peptides, nucleic acid sequences coding therefore, cells expressing such peptides or nucleic acids, and derivatives of such peptides, wherein the said therapeutic typically ameliorates or treats disease when administered in prophylactically or therapeutically effective dosages.

AGXT I340M therapeutics, of the invention include modifications, derivatives and analogs of AGXT I340M peptides, and nucleic acids encoding such peptides. In some embodiments, the AGXT I340M therapeutic, of the invention may be a peptide having a sequence of amino acids corresponding to the naturally-occurring wild-type AGXT I340M peptide as set out in SEQ ID NO: 2.

The AGXT I340M therapeutic, of the invention may include substantially purified compounds such as peptide fragments, modified peptide fragments, analogues or pharmacologically acceptable salts of AGXT I340M. Such compounds are collectively referred to herein as LPL S447X peptides. AGXT I340M peptides may include homologs of the AGXT I340M sequence, including homologs from species other than homo sapiens (which may have veterinary applications).

AGXT I340M peptides may include naturally occurring isoforms or genetic variants of AGXT I340M.

An AGXT I340M therapeutic is an AGXT I340M protein comprising an amino acid sequence having at least 90%, at least about 95%, at least about 97%, at least about 98%, at least about 99% or at least about 100% sequence identity to SEQ ID NO: 2 when optimally aligned over their entire length using the GAP program, and wherein the AGXT I340M protein comprises a methionine at a position corresponding to position 340 in SEQ ID NO: 2. A "position corresponding to a certain position in SEQ ID NO: 2" (e.g. 340) is herein understood as a position corresponding to that position in SEQ ID NO: 2 in an AGXT amino acid sequence other than SEQ ID NO: 2, when optimally aligned with SEQ ID NO: 2 over the entire length of using the GAP program.

In one embodiment the AGXT I340M protein is a protein having alanine:glyoxylate aminotransferase (AGXT; EC 2.6.1.44) activity as may be assayed as described by Rumsby et al (Ann Clin Biochem. 1997 Jul;34 ( Pt 4):400-4). In a preferred embodiment, the AGXT I340M protein is a protein having an amino acid sequence wherein active site residues, residues involved in substrate-binding, co-factor (pyridoxal 5'-phosphate) binding and/or subunit interaction are conserved. Such residues may be derived by comparison of the amino acid sequences of SEQ ID NO: 2 with the 3D-crystal structure of the human and *Anabaena* AGXT proteins as has been described by Zhang et al. (2003, J Mol Biol., 331(3):643-52) and Han et al. (2005, Proteins., 58(4):971-5), respectively), including identification of active site residues at positions 81 (S), 82 (G), 83 (H), 108 (W), 158 (S), 183 (D), 186 (A) 208 (Q) and 209 (K). According to Fold and Function Assignment System (FFAS; Jaroszewski L, Li W, Godzik A. 2002, Protein Sci, 11:1702-1713), the AGXT family has hundreds of homologous sequences in pro- and eukaryotic proteomes. Models for AGXT homologues can be accessed at http://www1.jcsg.org/cgi-bin/models/get mor.pl?key 17130350 . Sequence alignments of SEQ ID NO: 2 with these known AGXT amino acid sequences will indicate conserved regions and amino acid positions, the conservation of which are important for structure and enzymatic activity. These regions and positions will tolerate no or only conservative amino acid substitutions. Amino acid substitutions outside of these regions and positions may be unlikely to greatly affect AGXT activity.

In a preferred embodiment, the AGXT I340M therapeutic is an AGXT I340M protein comprising amino acid substitions selected from the group consisting of: a) a leucine at a position corresponding to position 11 in SEQ ID NO: 2; b) an arginine at a position corresponding to position 41 in SEQ ID NO: 2; c) an isoleucine at a position corresponding to position 152 in SEQ ID NO: 2; d) an arginine at a position corresponding to position 170 in SEQ ID NO: 2; e) a threonine at a position corresponding to position 244 in SEQ ID NO: 2; f) a threonine at a position corresponding to position 294 in SEQ ID NO: 2; g) an isoleucine at a position corresponding to position 326 in SEQ ID NO: 2; h) combinations of one or more of the substitutions amino acid b) to g); and, i) the combination of the substitutions a) and f). In a preferred ambodiment, the AGXT I340M therapeutic is an AGXT I340M protein selected from the group consisting of: a) an AGXT I340M protein comprising the amino acid sequence of SEQ ID NO: 2; and, b) an AGXT I340M protein comprising the amino acid sequence of SEQ ID NO: 2 and comprising amino acid substitions as defined in a) to i) above.

In another embodiment, a AGXT I340M therapeutic is a nucleic acid molecule comprising an nucleotide sequence encoding the AGXT I340M protein as defined herein above, or a virion of a viral gene therapy vector comprising the nucleic acid molecule.

In a preferred embodiment of the invention, a nucleotide sequence coding for the AGXT I340M protein has an improved codon usage bias for the human cell as compared to naturally occurring nucleotide sequence coding for the transferase.

The adaptiveness of a nucleotide sequence encoding the AGXT I340M protein to the codon usage of human cells may be expressed as codon adaptation index (CAI). A codon adaptation index is herein defined as a measurement of the relative adaptiveness of the codon usage of a gene towards the codon usage of highly expressed human genes. The relative adaptiveness (w) of each codon is the ratio of the usage of each codon, to that of the most abundant codon for the same amino acid. The CAI is defined as the geometric mean of these relative adaptiveness values. Non-synonymous codons and termination codons (dependent on genetic code) are excluded. CAI values range from 0 to 1, with higher values indicating a higher proportion of the most abundant codons (see Sharp and Li , 1987, Nucleic Acids Research 15: 1281-1295; also see: Kim et al., Gene. 1997, 199:293-301; zur Megede et al., Journal of Virology, 2000, 74: 2628-2635). Preferably, a nucleotide sequence encoding the AGXT I340M protein has a CAI of at least about 0.8, at least about 0.85, at least about 0.90, at least about 0.92, at least about 0.94, at least about 0.95, at least about 0.96 or at least about 0.97. The coding sequence may further be adapted for improved expression in the insect host cell by methods described in WO 2004/059556, and by modifying the CpG content of the coding sequence as described in WO 2006/015789. It is understood that such further adaptations may cause that not all codons in the nucleotide sequence coding for the common amino acid sequence in the first nucleotide sequence are common codons.

In one embodiment of invention, a nucleotide sequence encoding the AGXT I340M protein has the nucleotide sequence of SEQ ID NO: 1.

The invention also pertains to a nucleic acid construct comprising a nucleotide sequence encoding the AGXT I340M protein of the invention as herein defined above. In the nucleic acid construct the nucleotide sequence encoding the AGXT I340M protein preferably is operably linked to a mammalian cell-compatible expression control sequence, e.g., a promoter, preferably a mammalian cell-compatible promoter. Many such promoters are known in the art (see Sambrook and Russel, 2001, *supra*)*.* Constitutive promoters that are broadly expressed in many cell-types, such as the CMV promoter may be used. However, more preferred will be promoters that are inducible, tissue-specific, cell-type-specific, or cell cycle-specific. In a preferred embodiment a nucleotide sequence encoding the AGXT I340M protein is operably linked to a liver-specific promoter. Liver-specific promoters are particularly preferred for use in conjunction the non-erythroid deaminase.

Preferably, in a construct of the invention expression control sequences for liver-specific expression are e.g. selected from the group consisting of an α1-anti-trypsin (AAT) promoter, a thyroid hormone-binding globulin promoter, an albumin promoter, a thyroxin-binding globulin (TBG) promoter, an Hepatic Control Region (HCR)-ApoCII hybrid promoter, an HCR-hAAT hybrid promoter, an AAT promoter combined with the mouse albumin gene enhancer (Ealb) element and an apolipoprotein E promoter. Other examples include the E2F promoter for tumour-selective, and, in particular, neurological cell tumour-selective expression (Parr et al., 1997, Nat. Med. 3:1145-9) or the IL-2 promoter for use in mononuclear blood cells (Hagenbaugh et al., 1997, J Exp Med; 185: 2101-10). In a particularly preferred embodiment of the invention, the promoter may have the sequence of SEQ ID NO: 3. In another embodiment of the nucleic acid construct, a nucleotide sequence encoding the AGXT I340M protein is operably linked to a promoter for expression in human cells that is not a promoter from an AGXT gene, for example not a promoter from a human AGXT gene.

In a further preferred embodiment, the nucleic acid construct of the invention comprises a 3'UTR (or 3' non-translated sequence) downstream of the nucleotide sequence encoding the AGXT I340M protein. Suitable 3'UTR sequences are available to the skilled person. They may be derived from any mammalian and preferably human gene and will usually comprise a transcription termination site and a polyadenylation signal (such as e.g. AAUAAA or variants thereof). In a particularly preferred embodiment the nucleic acid construct comprises a 3'UTR derived from the human porphobilinogen deaminase gene such as e.g. SEQ ID NO: 4.

In another preferred embodiment of the nucleic acid construct of the invention, the expression control sequence that is operably linked to the nucleotide sequence encoding the AGXT I340M protein, is preceded upstream by a polyA insulator to terminate run-through transcription from possible upstream transcription units. A 3'UTR as described above and preferably at least comprising a transcription termination sequence may be used for this purpose. A preferred polyA insulator is a synthetic polyA insulator having the sequence of SEQ ID NO: 5.

In one preferred embodiment, the nucleic acid construct of the invention comprises a Kozak consensus sequence around the initiation codon of the nucleotide sequence encoding the AGXT I340M protein.

The Kozak consensus sequence is herein defined as GCCRCC(AUG)A (SEQ ID NO: 6), wherein R is a purine (i.e. A, adenosine or G, guanosine) and wherein (AUG) stands for the initiation codon of the AGXT I340M protein-coding sequence. In a preferred embodiment, the Kozak consensus sequence may be preceded by another GCC triplet.

The invention also provides a nucleic acid construct comprising a nucleotide sequence encoding the AGXT I340M protein that is operably linked to an expression control sequence as defined herein above, wherein the construct is an expression vector that is suitable for gene therapy of mammals, preferably gene therapy of humans. A preferred nucleic acid construct according to the invention is a viral gene therapy vector. Viral gene therapy vectors are well known in the art and e.g. include vectors based on an adenovirus, a parvovirus such as an adeno-associated virus (AAV), a herpes virus, a pox virus and a retrovirus. A preferred viral gene therapy vector is an AAV, adenoviral or a lentiviral vector.

Particularly preferred gene therapy vectors in the context of the present invention are parvoviral vectors. Thus, in this preferred aspect the invention relates the use of animal parvoviruses, in particular dependoviruses such as infectious human or simian AAV, and the components thereof (e.g., an animal parvovirus genome) for use as vectors for introduction and/or expression of the nucleotide sequences encoding a AGXT I340M protein in mammalian cells.

Viruses of the Parvoviridae family are small DNA animal viruses. The family Parvoviridae may be divided between two subfamilies: the Parvovirinae, which infect vertebrates, and the Densovirinae, which infect insects. Members of the subfamily Parvovirinae are herein referred to as the parvoviruses and include the genus Dependovirus. As may be deduced from the name of their genus, members of the Dependovirus are unique in that they usually require coinfection with a helper virus such as adenovirus or herpes virus for productive infection in cell culture. The genus Dependovirus includes AAV, which normally infects humans (e.g., serotypes 2, 3A, 3B, 5, and 6) or primates (e.g., serotypes 1 and 4, which are thought to have been originated from monkeys, but also infect humans), and related viruses that infect other warm-blooded animals (e.g., bovine, canine, equine, and ovine adeno-associated viruses).

Further information on AAV serotypes and on strategies for engineering hybrid AAV vectors derived from AAV serotypes is described in Wu et al. (2006, Molecular Therapy 14:316-327). For convenience the present invention is further exemplified and described herein by reference to AAV. It is however understood that the invention is not limited to AAV but may equally be applied to hybrid AAV vectors derived from two or more different AAV serotypes and to other parvoviruses and hybrids thereof.

The genomic organization of all known AAV serotypes is very similar. The genome of AAV is a linear, single-stranded DNA molecule that is less than about 5,000 nucleotides (nt) in length. Inverted terminal repeats (ITRs) flank the unique coding nucleotide sequences for the non-structural replication (Rep) proteins and the structural (VP) proteins. The VP proteins (VP1, -2 and -3) form the capsid. The terminal 145 nt are self-complementary and are organized so that an energetically stable intramolecular duplex forming a T-shaped hairpin may be formed. These hairpin structures function as an origin for viral DNA replication, serving as primers for the cellular DNA polymerase complex. Following wtAAV infection in mammalian cells the Rep genes (i.e. Rep78 and Rep52) are expressed from the P5 promoter and the P19 promoter, respectively and both Rep proteins have a function in the replication of the viral genome. A splicing event in the Rep ORF results in the expression of actually four Rep proteins (i.e. Rep78, Rep68, Rep52 and Rep40). However, it has been shown that the unspliced mRNA, encoding Rep78 and Rep52 proteins, in mammalian cells are sufficient for AAV vector production. Also in insect cells the Rep78 and Rep52 proteins suffice for AAV vector production.

A "recombinant parvoviral or AAV vector" (or "rAAV vector") herein refers to a vector comprising one or more polynucleotide sequences of interest, genes of interest or "transgenes" that are flanked by at least one parvoviral or AAV inverted terminal repeat sequences (ITRs). Such rAAV vectors can be replicated and packaged into infectious viral particles when present in an insect host cell that is expressing AAV rep and cap gene products (i.e. AAV Rep and Cap proteins). When an rAAV vector is incorporated into a larger nucleic acid construct (e.g. in a chromosome or in another vector such as a plasmid or baculovirus used for cloning or transfection), then the rAAV vector is typically referred to as a "pro-vector" which can be "rescued" by replication and encapsidation in the presence of AAV packaging functions and necessary helper functions.

Thus, the invention relates to a nucleic acid construct comprising a nucleotide sequence encoding an AGXT I340M protein as herein defined above, wherein the nucleic acid construct is a recombinant parvoviral or AAV vector and thus comprises at least one parvoviral or AAV ITR. Preferably, in the nucleic acid construct the nucleotide sequence encoding the AGXT I340M protein is flanked by parvoviral or AAV ITRs on either side. Any parvoviral or AAV ITR may be used in the constructs of the invention, including ITRs from AAV1, AAV2, AAV4, and/or AAV5. ITRs of AAV2 are most preferred. Examples of preferred ITR sequences for use in preferred nucleic acid constructs of the invention are given SEQ ID NO: 7 (left or upstream ITR) and SEQ ID NO: 8 (right or downstream ITR).

AAV is able to infect a number of mammalian cells. See, e.g., Tratschin et al. (1985, Mol. Cell Biol. 5:3251-3260) and Grimm et al. (1999, Hum. Gene Ther. 10:2445-2450). However, the cellular tropism of AAV differs among serotypes. See, e.g., Davidson et al. (2000, Proc. Natl. Acad. Sci. USA, 97:3428-3432), who discuss differences among AAV2, AAV4, and AAV5 with respect to mammalian CNS cell tropism and transduction efficiency and see Goncalves, 2005, Virol J. 2(1):43 who discusses approaches to modification of AAV tropism. For transduction of liver cells rAAV virions with AAV1, AAV8 and AAV5 capsid proteins are preferred (Nathwani et al., 2007, Blood 109(4): 1414-1421; Kitajima et al., 2006, Atherosclerosis 186(1):65-73), of which is rAAV virions with AAV5 or 8 capsid proteins are most preferred.

AAV sequences that may be used in the present invention for the production of recombinant AAV vectors in insect cells can be derived from the genome of any AAV serotype. Generally, the AAV serotypes have genomic sequences of significant homology at the amino acid and the nucleic acid levels, provide an identical set of genetic functions, produce virions which are essentially physically and functionally equivalent, and replicate and assemble by practically identical mechanisms. For the genomic sequence of the various AAV serotypes and an overview of the genomic similarities see e.g. GenBank Accession number U89790; GenBank Accession number J01901; GenBank Accession number AF043303; GenBank Accession number AF085716; Chlorini et al. (1997, J. Vir. 71: 6823-33); Srivastava et al. (1983, J. Vir. 45:555-64); Chlorini et al. (1999, J. Vir. 73:1309-1319); Rutledge et al. (1998, J. Vir. 72:309-319); and Wu et al. (2000, J. Vir. 74: 8635-47). AAV serotypes 1, 2, 3, 4 and 5 are preferred source of AAV nucleotide sequences for use in the context of the present invention. Preferably the AAV ITR sequences for use in the context of the present invention are derived from AAV1, AAV2, and/or AAV4. Likewise, the Rep (Rep78/68 and Rep52/40) coding sequences are preferably derived from AAV1, AAV2, and/or AAV4. The sequences coding for the VP1, VP2, and VP3 capsid proteins for use in the context of the present invention may however be taken from any of the known 42 serotypes, more preferably from AAV1, AAV2, AAV3, AAV4, AAV5, AAV6, AAV7, AAV8 or AAV9 or newly developed AAV-like particles obtained by e.g. capsid shuffling techniques and AAV capsid libraries.

AAV Rep and ITR sequences are particularly conserved among most serotypes. The Rep78 proteins of various AAV serotypes are e.g. more than 89% identical and the total nucleotide sequence identity at the genome level between AAV2, AAV3A, AAV3B, and AAV6 is around 82% (Bantel-Schaal et al., 1999, J. Virol., 73(2):939-947). Moreover, the Rep sequences and ITRs of many AAV serotypes are known to efficiently cross-complement (i.e., functionally substitute) corresponding sequences from other serotypes in production of AAV particles in mammalian cells. US2003148506 reports that AAV Rep and ITR sequences also efficiently cross-complement other AAV Rep and ITR sequences in insect cells.

The AAV VP proteins are known to determine the cellular tropism of the AAV virion. The VP protein-encoding sequences are significantly less conserved than Rep proteins and genes among different AAV serotypes. The ability of Rep and ITR sequences to cross-complement corresponding sequences of other serotypes allows for the production of pseudotyped rAAV particles comprising the capsid proteins of one serotype (e.g., AAV5) and the Rep and/or ITR sequences of another AAV serotype (e.g., AAV2). Such pseudotyped rAAV particles are a part of the present invention.

Modified "AAV" sequences also can be used in the context of the present invention, e.g. for the production of rAAV vectors in insect cells. Such modified sequences e.g. include sequences having at least about 70%, at least about 75%, at least about 80%, at least about 85%, at least about 90%, at least about 95%, or more nucleotide and/or amino acid sequence identity (e.g., a sequence having about 75-99% nucleotide sequence identity) to an AAV1, AAV2, AAV3, AAV4, AAV5, AAV6, AAV7, AAV8 or AAV9 ITR, Rep, or VP can be used in place of wild-type AAV ITR, Rep, or VP sequences.

Although similar to other AAV serotypes in many respects, AAV5 differs from other human and simian AAV serotypes more than other known human and simian serotypes. In view thereof, the production of rAAV5 can differ from production of other serotypes in insect cells. Where methods of the invention are employed to produce AAV5, it is preferred that one or more constructs comprising, collectively in the case of more than one construct, a nucleotide sequence comprising an AAV5 ITR, a nucleotide sequence comprises an AAV5 Rep coding sequence (i.e. a nucleotide sequence comprises an AAV5 Rep78). Such ITR and Rep sequences can be modified as desired to obtain efficient production of rAAV5 or pseudotyped rAAV5 vectors in insect cells. E.g., the start codon of the Rep sequences can be modified, VP splice sites can be modified or eliminated, and/or the VP1 start codon and nearby nucleotides can be modified to improve the production of rAAV5 vectors in the insect cell.

If an adenoviral vector is used in the invention, a preferred adenoviral vector may be modified to reduce the host response as reviewed by Russell (2000, J. Gen. Virol. 81: 2573-2604), or as described in US20080008690 and by Zaldumbide and Hoeben (Gene Therapy 2008:239-246).

The invention thus relates to a parvoviral virion comprising a nucleic acid construct as herein defined above, and parvoviral capsid protein as defined herein above.

The invention also provides a method for producing a recombinant parvoviral (rAAV) virion (comprising a recombinant parvoviral (rAAV) vector as defined above) in an insect cell. Preferably, the method comprises the steps of: (a) culturing an insect cell as defined in herein above under conditions such that recombinant parvoviral (rAAV) vector is produced; and, (b) recovery of the recombinant parvoviral (rAAV) vector. It is understood here that the recombinant parvoviral (rAAV) vector produced in the method preferably is an infectious parvoviral or AAV virion that comprise the recombinant parvoviral (rAAV) vector nucleic acids. Growing conditions for insect cells in culture, and production of heterologous products in insect cells in culture are well-known in the art and described e.g. in the above cited references on molecular engineering of insects cells. Preferred methods and constructs for the production of rAAV virions of the invention are disclosed in e.g. WO2007/046703 and WO2007/148971.

Preferably the methods for producing recombinant parvoviral virions further comprises the step of affinity-purification of the (virions comprising the) recombinant parvoviral (rAAV) vector using an anti-AAV antibody, preferably an immobilised antibody. The anti-AAV antibody preferably is a monoclonal antibody. A particularly suitable antibody is a single chain camelid antibody or a fragment thereof as e.g. obtainable from camels or llamas (see e.g. Muyldermans, 2001, Biotechnol. 74: 277-302). The antibody for affinity-purification of rAAV preferably is an antibody that specifically binds an epitope on a AAV capsid protein, whereby preferably the epitope is an epitope that is present on capsid protein of more than one AAV serotype. E.g. the antibody may be raised or selected on the basis of specific binding to AAV2 capsid but at the same time also it may also specifically bind to AAV1, AAV3 and AAV5 capsids.

The invention further pertains to an AGXT I340M therapeutic for use as a medicament or for use in a method of treatment. The AGXT I340M therapeutic for use as a medicament can be an AGXT I340M protein or nucleic acid molecule as herein defined above, or a virion of a gene therapy vector, such as e.g. a parvoviral virion as defined above.

Also, the invention pertains to an AGXT I340M therapeutic as herein defined for use in the treatment of an AGXT-responsive condition.

An AGXT-responsive condition can be a condition caused by a deficiency (for example a partial deficiency or complete deficiency (or substantially complete deficiency)) in alanine:glyoxylate aminotransferase. A deficiency in alanine:glyoxylate aminotransferase is understood to include absence (partial, complete or substantially complete) or subcellular mislocation of the alanine:glyoxylate aminotransferase. AGXT deficiency leads to a disease termed primary hyperoxaluria type I, which is also referred to as oxalosis I, glycolic aciduria, alanine-glyoxylate aminotransferase deficiency, and peroxisomal and/or hepatic alanine-glyoxylate aminotransferase deficiency. Accordingly, the invention pertains to treatment of primary hyperoxaluria type I. Treatment according to the invention includes treatment of one or more symptoms, such as amelioration thereof, of such an indication.

The invention also pertains to a pharmaceutical composition comprising an AGXT I340M therapeutic, a nucleic acid construct or a parvoviral virion as herein defined above. The pharmaceutical composition further preferably comprises a pharmaceutically acceptable carrier. Any suitable pharmaceutically acceptable carrier or excipient can be used in the present compositions (See e.g., Remington: The Science and Practice of Pharmacy, Alfonso R Gennaro (Editor) Mack Publishing Company, April 1997).

Preferred pharmaceutical forms would be in combination with sterile saline, dextrose solution, or buffered solution, or other pharmaceutically acceptable sterile fluids. Alternatively, a solid carrier, may be used such as, for example, microcarrier beads.

Also, the invention provides a method for treating an AGXT-responsive condition wherein the method comprises the step of administering an effective amount of an AGXT I340M therapeutic, a nucleic acid construct, a parvoviral virion or a pharmaceutical composition as defined herein to a subject with the AGXT-responsive condition. The AGXT-responsive condition can be a deficiency in alanine:glyoxylate aminotransferase such as e.g. primary hyperoxaluria type I.

In addition, the invention relation to use of an AGXT I340M therapeutic, a nucleic acid construct, a parvoviral virion or a pharmaceutical composition as defined herein in the manufacture of a medicament for use in the treatment of an AGXT-responsive condition.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The following examples are offered for illustrative purposes only, and are not intended to limit the scope of the present invention in any way.

### Examples

### 1. Example 1

The human AGXT cDNA wild type and several major and minor alleles and combination thereof were expressed with His-tags in E. *coli* (Top 10, Invitrogen). Expressed AGXT proteins were purified by metal-affinity chromatography according to standard procedures.

Purified protein was subject to SDS/PAGE analysis and Alanine-glyoxylate aminotransferase activity assay (Rumsby et al., Ann Clin Biochem. 1997 Jul;34 ( Pt 4):400-4.). Mean activity values (umoles/h/g total protein) of the AGXT proteins as expressed in E. *coli* are given in Table 1.

Table 1 Mean activity values (umoles/h/g total protein) of the AGXT proteins as expressed in E.coli. pTrcHis = empty vector, pTrcHisAGXT*WT = wild type AGXT (Genbank Acc. No. NM_000030); T indicates the I244T allele; M indicates the I340M allele; *L indicates the P11L allele; and R indicates the G170R allele. Combinations of these letter indicate proteins with the corresponding amino acid substitutions in one protein.

| CONSTRUCT | MEAN ACTIVITY | Standard Deviation |
|---|---|---|
| pTrcHis (Top10) | 0.370 | 0.169 |
| pTrcHisAGXT*WT | 36.351 | 11.735 |
| pTrcHisAGXT*T | 26.517 | 3.636 |
| pTrcHisAGXT*M | 45.920 | 8.399 |
| pTrcHisAGXT*L | 12.489 | 2.694 |
| pTrcHisAGXT*LM | 21.255 | 4.016 |
| pTrcHisAGXT*LR | 2.122 | 0.534 |
| pTrcHisAGXT*LRM | 8.617 | 1.289 |
| pTrcHisAGXT*LT | 0.923 | 0.319 |
| pTrcHisAGXT*LTM | 3.562 | 0.811 |

P11L is enough to promote a significant decrease in activity. Thus, the effect of the mutations depends significantly on the allelic background. Both G170R and I244T, in the minor haplotype, result in significant reduction of activity. The polymorphism I340M, when present in combination with either mutation, results in better residual activity. The expression of AGXT wild type with I340M (a variant rarely seen in the general population) results in a significantly higher activity than the most common major allele.

### 2. Example 2

### 2.1 Materials and methods

### 2.1.1 Animal experiments, urine and blood analyses

All animal experiments were approved by the institutional animal experimentation ethics committee, and carried out according to the Spanish and European law. B6;129SvAgxt^{tmIUII} mice were genotyped as described [Salido et al. PNAS 2006;103(48):18249-54. PMID: 17110443], bred and maintained in a pathogen-free facility, with free access to standard chow (A04, Safe, France) and water. Mice were placed in metabolic cages for single mouse (model 3600M, Tecniplast) and allowed to get acclimatized and used to a - 4 g/day powdered, oxalate-free diet (with <0.4 µmol/g oxalate, 0.5% Ca²⁺, and 0.25% Mg²⁺ (Harlan TD#01699, Indianapolis, Ind., USA,) during 3 days before the start of urine collection. Two 24 hr urine collections were performed each week in narrow tubes containing 50 µl 6 N HCl. The oxalate oxidase assay (http://www.greiner-diagnostic.com) was used to measure urine oxalate, while the Jaffe alkaline picrate test was used to measure urine creatinine. Twenty-four-hour urine samples that were <1 ml for 3 month old mice were typically seen in cages with signs of incomplete urine collection, and were excluded from the study. Similarly, samples with food or fecal contamination were excluded. At the end of the study, the mice were sacrificed, and blood was collected for biochemical analysis. Serum creatinine, BUN, GOT and GPT were measured in an autoanalyzer (A25, Biosystems, Spain). Serum samples were also used, diluted 1:100 in PBS, in Western blot analysis of recombinant human AGXT protein, to check for the presence of anti-AGXT mouse antibodies.

Liver samples were harvested for histology, enzyme assay, and northern and western blot analyses. Kidney samples were collected for histological analysis. Five males were selected for a more extensive histological study that included brain, lung, heart, stomach, liver, spleen, pancreas, kidney, testis and seminal vesicle. DNA and protein were also extracted from these tissues.

For gene therapy experiments, 12-16 week old mice were restrained and injected into the tail vein using a 1 ml syringe with 27G needle. AAV particles were dissolved in 5% sucrose-PBS solution.

### 2.1.2 Histological analysis

Tissues were fixed in 4% buffered paraformaldehyde and either embedded in paraffin or cryoprotected in 20% sucrose and snap-frozen in liquid nitrogen. Hematoxylin and eosin staining was done in all tissues, and calcium oxalate staining (Yasue, 1969 Acta Histochemica et Cytochemica 2:83-95) was performed on kidney sections. Kidney sections from a PH1 patient with nephrocalcinosis were used as positive control for the calcium oxalate staining. To measure the deposits, two random sections stained for CaOx were digitally imaged and processed with image analysis software (ImagePro). The threshold for positive areas was established automatically, and the area of the deposits was expressed as a percentage of the total digitized area.

### 2.1.3 AGT enzyme assay, Western blot and immunohistochemistry

AGT activity and western blot analyses were determined as described [Salido et al. PNAS 2006;103(48):18249-54. PMID: 17110443]. Immunohistochemistry was performed on frozen sections, incubating with 1:5000 rabbit anti-human AGXT antibody (a gift from Dr. C. Danpure) for 2 hrs, followed by 5 min. PBS washes, 3 times, and HRP-conjugated anti-rabbit serum (Dako), for 30 min. After another 3 PBS washes, a DAB-H2O2 solution was used as chromogen, and some sections were counterstained with hematoxylin. Control sections were also stained with 1:5000 anti-GS antibody (Santa Cruz Biotechnologies) to label perivenular zones.

### 2.1.4 Recombinant AAV construction, production and DNA analysis

The AAV plasmids used in this study contain the expression cassette flanked by two ITRs from the AAV2 and an appropriate stuffer sequence to adjust genome size to the optimal packaging capacity described for AAV. The expression cassette has the following elements: the 5'ITR from AAV2, a liver-specific promoter EalbAATp with regulatory sequences from the albumin enhancer (Kramer et al., 2003, Mol Ther. 7(3):375-85), either human AGXT cDNA (encoding the AGXT I340M variant - SEQ ID NO: 2) or enhanced GFP cDN A, the bovine growth hormone polyadenylation sequence, a woodchuck hepatitis virus posttranscriptional regulatory element (WPRE), and the 3'ITR from AAV2. Similar expression cassettes were also made without the WPRE sequence. The four AAV plasmids used in this study are named ssAAV-EalbAAT-AGT-polyA-WPRE, ssAAV-EalbAAT-AGT-polyA (expressing the therapeutic gene), ssAAV-EalbAAT-GFP-polyA-WPRE and ssAAV-EalbAAT-GFP-polyA (expressing the reporter gene eGFP).

AAV2/8 vectors were produced by calcium phosphate-mediated co-transfection in 293 cells of three different plasmids pAdDeltaF6, p5E18-VD2/8 and the therapeutic (AAV-polyA-EalbAAT-AGT-WPRE) or reporter gene (AAV-polyA-EalbAAT-GFP-WPRE), (Hermens et al, 1999, Human Gene Therapy,10:1885-91 and Gao et al 2002, Proc Natl Acad Sci USA 99:11854-9). Similarly, AAV2/5 vectors were produced using p5E18-VD2/5 instead. Briefly, 293 cells were co-transfected with pAdDeltaF6, either p5E18-VD2/8 or p5E18-VD2/5, and target vector by calcium phosphate and the virus was harvested by freeze thawing of the cells, 48h after transfection. The virus was purified by ion exchange column chromatography and iodixanol gradient centrifugation followed by filtration and further concentration against phosphate-buffered saline (PBS)-5% sucrose. Virus titers in terms of genome copies/ml were determined by Q-PCR performed in triplicate, using TaqMan (AppliedBiosystems) protocols, and primers pr300fw (5'-CCCTGTTTGCTCCTCCGATAA-3') and pr301rv (5'-GTCCGTATTTAAGCAGTGGATCCA-3'), which amplify a 95 bp fragment of the hAAT promoter region. Protein composition and purity was determined by sodium dodecyl sulfate-polyacrylamide gel electrophoresis (SDS-PAGE).

The number of AAV vector genomes per cell from different tissues was determined by real time PCR analysis with primers corresponding to the AGXT and WPRE sequences. Genomic DNA from different mouse tissues (liver, heart, brain, kidney, lung, spleen, testis and seminal vesicle) was isolated by proteinase K treatment and phenol extraction. About 100 ng of genomic DNA was used as template for PCR. Dilutions of the rAAV vector plasmid were used to generate a standard curve for determination of vector genome copies.

### 2.2 Results and discussion

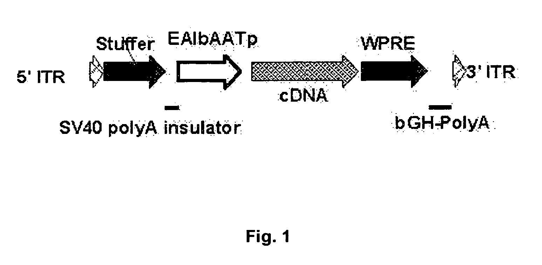

rAAV vectors were constructed by inserting the human *AGXT-c*DNA into an AAV2 vector plasmid, under the control of a hybrid EalbAAT liver-specific promoter. cDNAs were followed by a bGH polyadenylation signal and, in some constructs, WPRE post-transcriptional regulatory element (Figure 1).

The expression cassette was flanked by inverted terminal repeats from AAV2. AAV2/8 and AAV2/5 vectors were produced by calcium phosphate-mediated co-transfection in 293 cells of three different plasmids pAdDeltaF6, either p5E18-VD2/8 or p5E18-VD2/5 and the therapeutic (either AAV- EalbAAT-AGT- polyA-WPRE or AAV- EalbAAT-AGT- polyA) or reporter gene (either AAV- EalbAAT-GFP- polyA-WPRE or AAV- EalbAAT-GFP- polyA), (Hermens et al, 1999 and Gao et al, 2002 Proc Natl Acad Sci USA 99:11854-9).

In a first set of experiments, we used AAV8 vectors at doses of 5x10¹² viral particles/kg body weight, aimed to transduce most of the hepatocytes, in male Agxt1KO mice (n= 10 animals per group). Our goal was a sustained correction of hyperoxaluria in a 50 day follow-up. In addition, we wanted to test the functional reserve provided by gene transfer after a challenge with ethylene glycol, a glyoxylate precursor.

Mice were placed in metabolic cages, fed oxalate-free diet and water ad-libitum, and allowed to adjust for 3 days. Basal oxalate excretion was measured in 24 hr. urine, collected in acidified tubes, using an oxalate oxidase-based kit. Creatinine concentration was also measured by the alkaline picric acid method. Mean daily oxalate excretion was 2.06±0.74 µmoles/24hrs. Purified, high titer (1.7-6.3 x10¹¹ viral particles / ml) preparations of rAAV2-AGXT-2/8 vectors were administered to adult Agxt1KO mice, 12-16 weeks old, by single tail vein injections at a dose of 5x10¹² viral particles /kg body weight, in 0.2 ml PBS-sucrose. An additional group of 5 *Agxt*^{+/+} males, not injected with any vector, were used as normal controls.

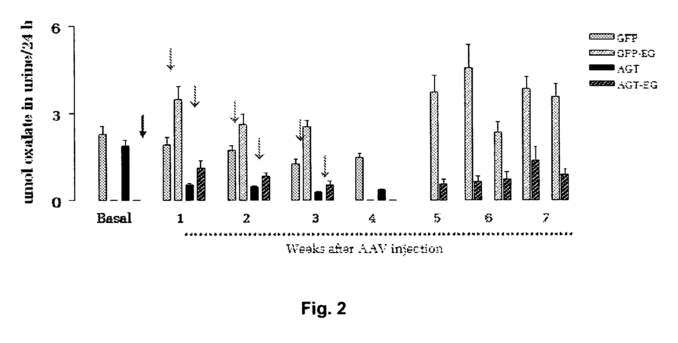

Urine oxalate excretion was followed during a total 50 day observation period, Figure 2 shows the mean oxalate excretion for animals injected with either the human AGXT cDNA expressing vector or the GFP expressing control. During the first two weeks of the study, mice were challenged with 0.25 ml of 0.5M ethylene glycol (EG) in three occasions, by gavage, to follow the response to a discrete overload of the glyoxylate pathway. Both basal oxaluria and the increase in oxalate levels following the administration of the glyoxylate precursor (EG) were significantly lower in the treated group, compared with the controls, as evidenced after only 6 days post-injection, bringing urine oxalate levels in the treated animals 4 weeks after injection down to the range normally observed in wild type mice (0.37±0.11 vs. 0.31±0.13 µmoles/24hr, respectively, p=0.0.28).

The response to the administration of 0.25 ml of 0.5M ethylene glycol was significantly blunted in mice treated with AGXT-AAV8 compared to those receiving GFP-AAV8 (0.34±0.37 µmol/24hrs vs. 1.24±0.57 µmol/24hrs, respectively, p= 0.001). After 4 weeks, 0.5% EG was included in the drinking water, and the increase in oxalate excretion was significantly smaller in AGXT-AAV8 treated animals than in controls injected with GFP-AAV8 (increase of 0.18±0.34 vs. 2.11±1.31 µmoles/24hr, respectively, p<0.001) and even smaller than in wild type mice subjected to the same challenge (0.50±0.21 µmoles/24hr, p= 0.005). These data are consistent with AGXT-AAV8 injection accounting for a larger functional reserve in treated mice than even wild type animals. During the last two weeks of the experiment, mice were subjected to 0.7% EG in drinking water, which resulted in significantly smaller increases of oxaluria in AGXT-AAV8 treated animals than in controls injected with GFP-AAV8 (increase of 0.54±0.56 vs. 2.46±0.7 µmoles/24hr, respectively, p=0.000), a response not significantly different from the one observed in wild type controls drinking 0.7% ethylene glycol (increase of 0.92±0.33, p=0.06).

During the study period, three animals in the control group died with pathological finding of nephrocalcinosis (see below). None of the AAV8-AGXT threated mice died. At the end of the seventh week of study, mice were sacrificed and tissues and blood were collected. All kidneys from the AAV8-GFP injected mice, but none from the mice that received AAV8-AGXT, exhibited some degree of nephrocalcinosis at the end of the study. Four mice showed few calcium oxalate (CaOx) deposits in the medullary region only; three mice presented moderate CaOx deposits in the cortex and medulla; and in the three mice that died there was severe and widespread nephrocalcinosis. Plasma could not be obtained from the mice that died prematurely, but blood urea nitrogen (BUN) concentrations were significantly elevated among the remaining seven AAV8-BUN treated mice compared to those that received AAV8-AGXT (26.7±9.9 vs 19.1±1-3 mg/dl, respectively, p=0.04).

Hepatic AGT enzyme activity and protein, by western blot and immunohistochemistry, were determined in all sacrificed animal at the end of the seventh week after injection. AGT activity was significantly higher in *Agxt*^{-/-} mice injected with AGXT-AAV2/8 than in the GFP-AAV2/8 controls (24.9±10.6 vs. 4.1±1.5 nmole/min*mg protein, p=0.002). Significant residual activity is present in liver extracts of *Agxt^{-l-}* animals, mainly with alanine as a substrate (AGT activity), but also when serine was used as a substrate (SGT activity) [Salido et al. "Alanine-glyoxylate aminotransferase-deficient mice, a model for primary hyperoxaluria that responds to adenoviral gene transfer." Proc.Natl.Acad.Sci.U.S.A 103.48 (2006): 18249-54].

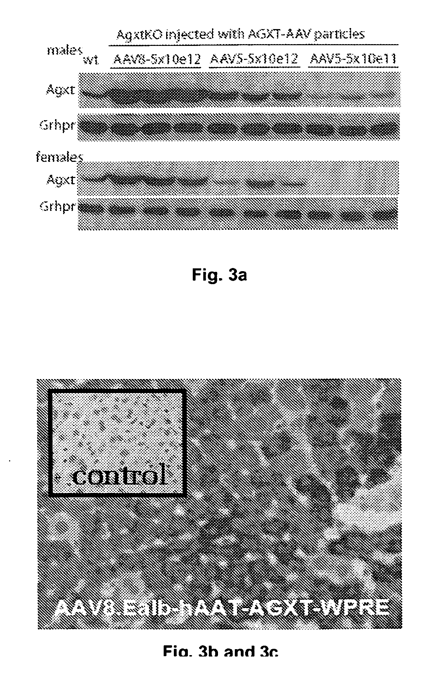

Western analysis of AGT protein in liver, as shown in Figure 3a, revealed robust AGT expression in rAAV-treated mice (lanes 2 to 10) compared to wild-type (lane 1) while no protein could be detected in control Agxt1KO mice treated with GFP-AAV2/8.

Transduction rates and expression of AGXT-AAV2/8 in different tissues of treated animals was determined by western blot. Brain, lung, spleen, kidney, seminal vesicles and testicles did not contain detectable AGT protein, but heart samples consistently showed low levels of AGT protein, evident after longer exposures of film (data not shown). The presence of AGXT-AAV2/8 DNA in various tissues was evaluated by PCR, using primers annealing to the 3' region of AGXT coding sequence and the WPRE sequence. Standard curves were made with AGXT-AAV8 and mouse interleukin 2 (mIL2) plasmids, and variations in the amount of template DNA were corrected by running parallel amplifications of the mIL2 gene. In livers of AGXT-AAV8 treated mice, we found 899±74 vector copies per diploid genome with the real-time PCR analysis. The estimation of vector copy number per mouse genome in other tissues were: 4.4±0.8 (kidney), 1.6±0.6 (lung), 0.7±0.1 (spleen), 1.3±0.1 (testis), and 3.3±0.6 (heart). Thus, two months after injection of 5 x10¹² viral particles per mouse, close to a thousand rAAV copies per transduced hepatocyte are detected, similar to what others have reported for this type of vectors [Nakai H, Fuess S, Storm TA, Muramatsu S, Nara Y, Kay MA: Unrestricted hepatocyte transduction with adeno-associated virus serotype 8 vectors in mice. J Virol. 2005 79(1):214-24.PMID: 15596817].

By immunohistochemistry, using rabbit anti-AGT antibody, striking differences could be seen between livers from Agxt1KO mice treated with either AGXT-AAV2/8 or GFP-AAV2/8 (Figure 3b). Indeed, most of the hepatocytes from mice injected with AGXT-AAV2/8 particles showed some degree of immunostaining, with a characteristic cytoplasmic granular pattern (Figure 3c) while no AGT staining was observed in control mice injected with eGFP-AAV2/8, which, conversely, showed abundant green fluorescence in liver tissue sections under the UV microscope (not shown).

Primary cultures of isolated hepatocytes, performed in one mouse from each group, were used to ascertain the subcellular localization of the expressed AGT protein by confocal microscopy (data not shown). Intense AGT immunofluorescence was observed in the peroxisomes of AGXT-AAV2/8 treated hepatocytes, showing colocalization with peroxisomal protein PMP70, but not with the mitochondrial marker (mitotracker).

Serum transaminases (alanine aminotransferase ALT/GPT and aspartate aminotransferase AST/GOT) were not elevated (58.7±6.2 and 39±6.9 U/L, vs. 56.3±10.8 and 37.9±4.1 U/L, respectively, in non-treated mice), and no liver lesions were detected on H&E sections. This is in agreement with the lack of liver damage or toxicity reported by others using this type of rAAV vector at similar doses [Nakai H, Fuess S, Storm TA, Muramatsu S, Nara Y, Kay MA: Unrestricted hepatocyte transduction with adeno-associated virus serotype 8 vectors in mice. J Virol. 2005 799(1):214-24.PMID: 15596817], [Gregorevic P, Blankinship MJ, Allen JM, Crawford RW, Meuse L, Miller DG,Russell DW, Chamberlain JS: Systemic delivery of genes to striated muscles using adeno-associated viral vectors. Nat Med. 2004 Aug;10(8):828-34. Epub 2004 Jul 25. PMID: 15273747]. In addition, we did not detect any significant titer of AGXT antibody, by western blot, in serum samples of AGXT-AAV2/8 treated mice.

We carefully inspected all the livers of our mice, including histological analysis, and found no evidence of oncogenesis after 50 day follow-up, in agreement with a large long-term study that found no evidence for tumorigenesis in AAV-treated mice [Bell et al. "No evidence for tumorigenesis of AAV vectors in a large-scale study in mice." Mol.Ther. 12.2 (2005): 299-306].

Previous studies have shown that AAV5 is a better vector for the transduction of human liver. In a second set of experiments, we used several doses of AAV5 vectors, devoid of WPRE sequence, at either 1.5x10¹³, 5x10¹², or 5x10¹¹ viral particles /kg body weight, and AAV8 vectors, with WPRE, at 5x10¹² viral particles/ kg, in both female and male Agxt1KO mice (n= 5 animals per group). The goal of this set of experiments was to compare the effect of gene transfer with AAV5 with respect to the results achieved with the serotype capable of transducing the most hepatocytes in mice, namely AAV8. We also wanted to evaluate possible gender differences in the response. Agxt1KO mice were placed in metabolic cages, fed oxalate-free diet and water *ad-libitum,* allowed to adjust for 3 days, and oxalate excretion was measured in 24 hr. urine.

Purified, high titer (4.5-10 x10¹¹ viral particles/ml) preparations of rAAV2-AGXT-2/5 and -2/8 vectors, as well as rAAV2-eGFP-2/5 vectors, were administered to adult Agxt1KO mice, 12-16 weeks old, by single tail vein injections in 0.2-0.3 ml PBS-sucrose.

Urine oxalate excretion was followed during a total 35 day observation period, Figure 4 shows the mean oxalate excretion for animals injected with either the human AGXT cDNA expressing vector or the GFP expressing control, at various doses of AAV5. All AGXT vector dose tested induced a significant reduction of oxalate excretion, compared with GFP controls. During the last week of the study, male mice were challenged with 0.5% ethylene glycol (EG) in drinking water, to follow the response to an overload of the glyoxylate cycle. The increases in oxalate excretion following the administration of the glyoxylate precursor were significantly lower in the groups receiving AGXT-AAV8 (0.44±0.49 µmole/24hrs) and the highest dose of AGXT-AAV5 (1.41±1.09 µmole/24hrs) than in those injected with GFP-AAV5 (4.49±1.41 µmole/24hrs) (p=0.008 in both pairwise comparisons). On the other hand, the increases in oxalate excretion following the administration after the challenge of mice that received AGXT-AAV8 and the highest dose of AGXT-AAV5 were not significantly different from those observed in wild type mice (0.55±0.24 µmole/24hrs).

These results are consistent with the analysis of AGXT expression in the liver at the end of the study. Both AGT activity and western blot analysis showed higher levels of expression when using AAV8 vectors, compared with AAV5 vectors, both in males and females. Nevertheless, the levels of expression achieved with 1.5x10¹³ AAV5 particles / Kg body weight were consistently higher than those observed in wild type mice. Lower doses (5x10¹² and 5x10¹¹ AAV5 particles/ kg) still resulted in significant AGT expression in male livers, while these doses resulted in relatively low signal in female livers.

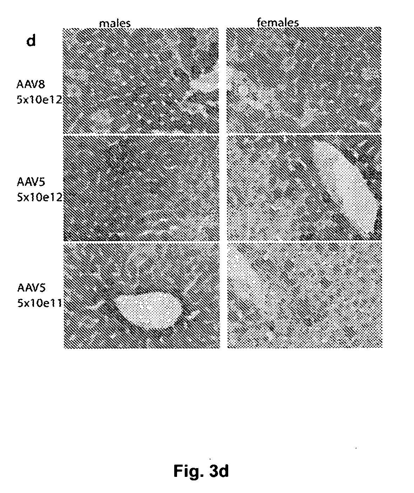

Parallel results were obtained by immunohistochemical staining of frozen liver sections, with percentages of hepatocytes transduced around 90% for AAV8 and the highest dose of AAV5, 60% for 5x10¹² AAV5 particles/kg, and down to 10% (and less than 1% in females) for 5x10¹¹ AAV5 particles/kg (Fig. 3d).

Our experiments establish that gene transfer with vectors capable of transducing a large proportion of hepatocytes, such as AAV2/8 and rAAV2/5, is a promising new therapy for primary hyperoxaluria type I. In this type of disease, where non-transduced hepatocytes would continue to produce oxalate, the therapeutic goal can only be achieved if enough hepatocytes are transduced so that the overall production of oxalate by the treated liver remains well below the capacity of the kidneys to excrete oxalate. The relatively large glomerular filtration rate (GFR) of the mouse, estimated at around 13 ml/min Kg body weight [Meneton P, Ichikawa I, Inagami T, Schnermann J. Renal physiology of the mouse.Am J Physiol Renal Physiol. 2000 Mar;278(3):F339-51. Review. PMID: 10710537], might account for the lack of nephrocalcinosis in Agxt1KO mice, unless they are challenged with glyoxylate precursors. The administration of ethylene glycol, at doses that do not produce renal injury in the mouse, increases the oxalate excretion to levels leading to nephrocalcinosis in Agxt1KO mice, but not in wild type controls [Salido EC, Li XM, Lu Y, Wang X, Santana A, Roy-Chowdhury N, Torres A, Shapiro LJ, Roy-Chowdhury J. Alanine-glyoxylate aminotransferase-deficient mice, a model for primary hyperoxaluria that responds to adenoviral gene transfer. Proc Natl Acad Sci USA. 2006;103(48):18249-54]. Both rAAV2/8 and rAAV2/5, at doses above 5x10¹¹ viral particles/ kg body weight are able to blunt the increase observed in mice drinking 0.5% ethylene glycol to levels similar to those observed in wild type mice. Similar results (not shown) were obtained with mice fed 0.5% glycolate in the diet.
Although AAV8 and high doses of AAV5 seem to correct the oxalate excretion similarly in males and females, substantially worse corrections were achieved in female mice injected with the lower doses of AAV5 constructs. Gender differences in expression of AAV vectors have been reported previously [Davidoff AM, Ng CY, Zhou J, Spence Y, Nathwani AC. Sex significantly influences transduction of murine liver by recombinant adeno-associated viral vectors through an androgen-dependent pathway Blood. 2003 Jul 15;102(2):480-8; Pañeda A, Vanrell L, Mauleon I, Crettaz JS, Berraondo P, Timmermans EJ, Beattie SG, Twisk J, van Deventer S, Prieto J, Fontanellas A, Rodriguez-Pena MS and Gonzalez-Aseguinolaza G. Effect of AAV serotype and genome structure over liver transduction and biodistribution in mice of both genders. Hum Gene Ther 2009, 20(8):908-17], but little is known about the mechanisms involved.

In summary, we showed that a single tail vein administration of rAAV2-AGXT-2/5 or 2/8 resulted in sustained correction of the PH1 mouse phenotype, without evidence of liver damage or toxicity.

This AAV-based gene transfer approach resulted in liver expression of the human AGXT cDNA sufficiently robust as to overcome the potential problem posed by the continued oxalate production of non-transduced hepatocytes. We also demonstrated that the product of the human AGXT gene is correctly targeted to the peroxisome in the mouse hepatocyte, and it complements the deficit of the mouse Agxt1KO model.

## Claims

1. An AGXT I340M therapeutic for use as a medicament, wherein the AGXT I340M therapeutic is selected from the group consisting of:
a) an AGXT I340M protein comprising an amino acid sequence having at least 90% sequence identity to SEQ ID NO: 2 when optimally aligned over their entire length using the GAP program, and wherein the AGXT I340M protein comprises a methionine at a position corresponding to position 340 in SEQ ID NO: 2;
b) a nucleic acid molecule comprising an nucleotide sequence encoding an AGXT I340M protein as defined in a); and,
c) a virion of a viral gene therapy vector comprising a nucleic acid molecule as defined in b).

2. An AGXT I340M therapeutic for use in the treatment of primary hyperoxaluria type I, wherein the AGXT I340M therapeutic is selected from the group consisting of:
a) an AGXT I340M protein comprising an amino acid sequence having at least 90% sequence identity to SEQ ID NO: 2 when optimally aligned over their entire length using the GAP program, and wherein the AGXT I340M protein comprises a methionine at a position corresponding to position 340 in SEQ ID NO: 2;
b) a nucleic acid molecule comprising an nucleotide sequence encoding an AGXT I340M protein as defined in a); and,
c) a virion of a viral gene therapy vector comprising a nucleic acid molecule as defined in b).

3. Use of an AGXT I340M therapeutic for the manufacture of a medicament for treatment of primary hyperoxaluria type I, wherein the AGXT I340M therapeutic is selected from the group consisting of:
a) an AGXT I340M protein comprising an amino acid sequence having at least 90% sequence identity to SEQ ID NO: 2 when optimally aligned over their entire length using the GAP program, and wherein the AGXT I340M protein comprises a methionine at a position corresponding to position 340 in SEQ ID NO: 2;
b) a nucleic acid molecule comprising an nucleotide sequence encoding an AGXT I340M protein as defined in a); and,
c) a virion of a viral gene therapy vector comprising a nucleic acid molecule as defined in b).

4. An AGXT I340M therapeutic for use according to claim 1 or claim 2 or a use according to claim 3, wherein the AGXT I340M protein comprises amino acid substitutions selected from the group consisting of:
a) a leucine at a position corresponding to position I in SEQ ID NO: 2;
b) an arginine at a position corresponding to position 41 in SEQ ID NO: 2;
c) an isoleucine at a position corresponding to position 152 in SEQ ID NO: 2;
d) an arginine at a position corresponding to position 170 in SEQ ID NO: 2;
e) a threonine at a position corresponding to position 244 in SEQ ID NO: 2;
f) a threonine at a position corresponding to position 294 in SEQ ID NO: 2;
g) an isoleucine at a position corresponding to position 326 in SEQ ID NO: 2
h) combinations of one or more of the substitutions amino acid b) to g); and,
i) the combination of the substitutions a) and f).

5. An AGXT I340M therapeutic for use according to claim 1 or claim 2 or a use according to claim 3, wherein the AGXT I340M protein is selected from the group consisting of:
a) an AGXT I340M protein comprising the amino acid sequence of SEQ ID NO: 2; and,
b) an AGXT I340M protein comprising the amino acid sequence of SEQ ID NO: 2 and comprising one or more amino acid substitutions as defined in claim 4.

6. An AGXT I340M therapeutic for use according to claim 1 or claim 2 or a use according to claim 3, wherein the AGXT I340M therapeutic is a nucleic acid molecule comprising a nucleotide sequence encoding an AGXT I340M protein as defined in claim 4 or 5.

7. A nucleic acid construct comprising a nucleotide sequence coding for a AGXT I340M protein as defined in any one of claims 1 to 6, wherein the nucleotide sequence is operably linked to a promoter for expression in human cells and, optionally, wherein the promoter is not a promoter from a human AGXT gene.

8. A nucleic acid construction according to claim 7, wherein the promoter is a liver-specific promoter.

9. A nucleic acid construct according to claim 8, wherein the liver-specific promoter is selected from the group consisting of an α1-anti-trypsin (AAT) promoter, a thyroid hormone-binding globulin promoter, an albumin promoter, a thyroxin-binding globulin (TBG) promoter, an Hepatic Control Region (HCR)-ApoCII hybrid promoter, an HCR-hAAT hybrid promoter, an AAT promoter combined with the mouse albumin gene enhancer (Ealb) element and an apolipoprotein E promoter.

10. A nucleic acid construct according to claim 7, wherein the promoter has the sequence of SEQ ID NO: 3.

11. A nucleic acid construct according to any one of claims 7 to 10, wherein the construct is a viral gene therapy vector, preferably a parvoviral vector.

12. A parvoviral virion comprising a nucleic acid construct as defined in any one of claims 7 to 11.

13. A pharmaceutical composition comprising an AGXT I340M therapeutic for use as defined in any one of claims 1 to 6, a nucleic acid construct as defined in any one of claims 7 to 11 or a parvoviral virion as defined in claim 12 and a pharmaceutically acceptable carrier.

14. A nucleic acid construct as defined in any one of claims 7 to 11, a parvoviral virion as defined in claim 12 or a pharmaceutical composition as defined in claim 13 for use in the treatment of primary hyperoxularia type I.

15. Use of a nucleic acid construct as defined in any one of claims 7 to 11, a parvoviral virion as defined in claim 12 or a pharmaceutical composition as defined in claim 13 in the manufacture of a medicament for use in the treatment of primary hyperoxaluria type I.

## Patentansprüche

1. AGXT 1340M-Therapeutikum zur Verwendung als Medikament, wobei das AGXT 1340M-Therapeutikum aus der Gruppe, bestehend aus:
a) einem AGXT I340M-Protein, das eine Aminosäuresequenz umfasst, die mindestens 90 % Sequenzidentität mit SEQ ID NO: 2 aufweist, wenn sie über ihre gesamte Länge mittels des GAP-Programms optimal angeglichen sind, und wobei das AGXT 1340M-Protein ein Methionin an einer Position umfasst, die der Position 340 in SEQ ID NO: 2 entspricht,
b) einem Nukleinsäuremolekül, das eine Nukleotidsequenz umfasst, die ein AGXT 1340M-Protein kodiert, wie es in a) definiert ist, und
c) einem Virion eines viralen Gentherapievektors, das ein Nukleinsäuremolekül umfasst, wie es in b) definiert ist,
ausgewählt ist.

2. AGXT 1340M-Therapeutikum zur Verwendung bei der Behandlung von primärer Hyperoxalurie Typ I, wobei das AGXT 1340M-Therapeutikum aus der Gruppe, bestehend aus:
a) einem AGXT I340M-Protein, das eine Aminosäuresequenz umfasst, die mindestens 90 % Sequenzidentität mit SEQ ID NO: 2 aufweist, wenn sie über ihre gesamte Länge mittels des GAP-Programms optimal angeglichen sind, und wobei das AGXT 1340M-Protein ein Methionin an einer Position umfasst, die der Position 340 in SEQ ID NO: 2 entspricht,
b) einem Nukleinsäuremolekül, das eine Nukleotidsequenz umfasst, die ein AGXT 1340M-Protein kodiert, wie es in a) definiert ist, und
c) einem Virion eines viralen Gentherapievektors, das ein Nukleinsäuremolekül umfasst, wie es in b) definiert ist,
ausgewählt ist.

3. Verwendung eines AGXT 1340M-Therapeutikums zur Herstellung eines Medikaments zur Behandlung von primärer Hyperoxalurie Typ I, wobei das AGXT 1340M-Therapeutikum aus der Gruppe, bestehend aus:
a) einem AGXT I340M-Protein, das eine Aminosäuresequenz umfasst, die mindestens 90 % Sequenzidentität mit SEQ ID NO: 2 aufweist, wenn sie über ihre gesamte Länge mittels des GAP-Programms optimal angeglichen sind, und wobei das AGXT 1340M-Protein ein Methionin an einer Position umfasst, die der Position 340 in SEQ ID NO: 2 entspricht,
b) einem Nukleinsäuremolekül, das eine Nukleotidsequenz umfasst, die ein AGXT 1340M-Protein kodiert, wie es in a) definiert ist, und
c) einem Virion eines viralen Gentherapievektors, das ein Nukleinsäuremolekül umfasst, wie es in b) definiert ist,
ausgewählt ist.

4. AGXT 1340M-Therapeutikum zur Verwendung nach Anspruch 1 oder Anspruch 2 oder Verwendung nach Anspruch 3, wobei das AGXT 1340M-Protein Aminosäuresubstitutionen umfasst, die aus der Gruppe, bestehend aus
a) einem Leucin an einer Position, die der Position 11 in SEQ ID NO: 2 entspricht,
b) einem Arginin an einer Position, die der Position 41 in SEQ ID NO: 2 entspricht,
c) einem Isoleucin an einer Position, die der Position 152 in SEQ ID NO: 2 entspricht,
d) einem Arginin an einer Position, die der Position 170 in SEQ ID NO: 2 entspricht,
e) einem Threonin an einer Position, die der Position 244 in SEQ ID NO: 2 entspricht,
f) einem Threonin an einer Position, die der Position 294 in SEQ ID NO: 2 entspricht,
g) einem Isoleucin an einer Position, die der Position 326 in SEQ ID NO: 2 entspricht,
h) Kombinationen von einer oder mehreren der Substitutionsaminosäure b) bis g) und
i) der Kombination der Substitutionen a) und f),
ausgewählt sind.

5. AGXT 1340M-Therapeutikum zur Verwendung nach Anspruch 1 oder Anspruch 2 oder Verwendung nach Anspruch 3, wobei das AGXT 1340M-Protein aus der Gruppe, bestehend aus
a) einem AGXT I340M-Protein, das die Aminosäuresequenz von SEQ ID NO: 2 umfasst und
b) einem AGXT I340M-Protein, das die Aminosäuresequenz von SEQ ID NO: 2 umfasst und eine oder mehrere Aminosäuresubstitution(en), die in Anspruch 4 definiert ist oder sind, umfasst,
ausgewählt ist.

6. AGXT 1340M-Therapeutikum zur Verwendung nach Anspruch 1 oder Anspruch 2 oder Verwendung nach Anspruch 3, wobei das AGXT 1340M-Therapeutikum ein Nukleinsäuremolekül ist, das eine Nukleotidsequenz umfasst, die ein AGXT 1340M-Protein kodiert, wie es in Anspruch 4 oder 5 definiert ist.

7. Nukleinsäurekonstrukt, das eine Nukleotidsequenz umfasst, die ein AGXT 1340M-Protein kodiert, wie es in einem der Ansprüche 1 bis 6 definiert ist, wobei die Nukleotidsequenz funktionell mit einem Promotor zur Expression in menschlichen Zellen verbunden ist und wobei gegebenenfalls der Promotor nicht ein Promotor von einem menschlichen AGXT-Gen ist.

8. Nukleinsäurekonstruktion nach Anspruch 7, wobei der Promotor ein Leberspezifischer Promotor ist.

9. Nukleinsäurekonstrukt nach Anspruch 8, wobei der Leber-spezifische Promotor aus der Gruppe, bestehend aus einem α1-Anti-Trypsin (AAT)-Promotor, einem Thyroidhormonbindenden Globulin-Promotor, einem Albumin-Promotor, einem Thyroxin-bindenden Globulin (TBG)-Promotor, einem hepatische Kontrollregion (HCR)-ApoCII-Hybrid-Promotor, einem HCR-hAAT-Hybrid-Promotor, einem AAT-Promotor, der mit dem Maus-Albumingenverstärker (Ealb)-Element kombiniert ist, und einem Apolipoprotein E-Promotor, ausgewählt ist.

10. Nukleinsäurekonstrukt nach Anspruch 7, wobei der Promotor die Sequenz von SEQ ID NO: 3 aufweist.

11. Nukleinsäurekonstrukt nach einem der Ansprüche 7 bis 10, wobei das Konstrukt ein viraler Gentherapievektor, vorzugsweise ein parvoviraler Vektor ist.

12. Parvovirales Virion, das ein Nukleinsäurekonstrukt umfasst, das in einem der Ansprüche 7 bis 11 definiert ist.

13. Pharmazeutische Zusammensetzung, die ein AGXT 1340M-Therapeutikum zur Verwendung gemäß der Definition in einem der Ansprüche 1 bis 6, ein Nukleinsäurekonstrukt gemäß der Definition in einem der Ansprüche 7 bis 11 oder ein parvovirales Virion gemäß der Definition in Anspruch 12 und einen pharmazeutisch verträglichen Träger umfasst.

14. Nukleinsäurekonstrukt gemäß der Definition in einem der Ansprüche 7 bis 11, parvovirales Virion gemäß der Definition in Anspruch 12 oder pharmazeutische Zusammensetzung gemäß der Definition in Anspruch 13 zur Verwendung bei der Behandlung von primärer Hyperoxularie Typ I.

15. Verwendung eines Nukleinsäurekonstrukts gemäß der Definition in einem der Ansprüche 7 bis 11, eines parvoviralen Virions gemäß der Definition in Anspruch 12 oder einer pharmazeutischen Zusammensetzung gemäß der Definition in Anspruch 13 bei der Herstellung eines Medikaments zur Verwendung bei der Behandlung von primärer Hyperoxalurie Typ I.

## Revendications

1. Agent thérapeutique AGXT I340M destiné à être utilisé en tant que médicament, dans lequel l'agent thérapeutique AGXT I340M est sélectionné dans le groupe constitué par :
a) une protéine AGXT I340M comprenant une séquence d'acides aminés ayant au moins 90 % d'identité de séquence avec SEQ ID N° : 2 lorsqu'elles sont alignées de manière optimale sur toute leur longueur à l'aide du programme GAP, et où la protéine AGXT I340M comprend une méthionine dans une position correspondant à la position 340 du SEQ ID N° : 2 ;
b) une molécule d'acide nucléique comprenant une séquence nucléotidique codant pour une protéine AGXT I340M telle qu'elle est définie en a); et
c) un virion d'un vecteur viral de thérapie génique comprenant une molécule d'acide nucléique telle qu'elle est définie en b).

2. Agent thérapeutique AGXT I340M destiné à être utilisé dans le traitement de l'hyperoxalurie primaire de type I, dans lequel l'agent thérapeutique AGXT I340M est sélectionné dans le groupe constitué par :
a) une protéine AGXT I340M comprenant une séquence d'acides aminés ayant au moins 90 % d'identité de séquence avec SEQ ID N° : 2 lorsqu'elles sont alignées de manière optimale sur toute leur longueur à l'aide du programme GAP, et où la protéine AGXT I340M comprend une méthionine dans une position correspondant à la position 340 du SEQ ID N° : 2 ;
b) une molécule d'acide nucléique comprenant une séquence nucléotidique codant pour une protéine AGXT I340M telle qu'elle est définie en a); et
c) un virion d'un vecteur viral de thérapie génique comprenant une molécule d'acide nucléique telle qu'elle est définie en b).

3. Utilisation d'un agent thérapeutique AGXT I340M pour la fabrication d'un médicament destiné au traitement de l'hyperoxalurie primaire de type I, dans laquelle l'agent thérapeutique AGXT I340M est sélectionné dans le groupe constitué par :
a) une protéine AGXT I340M comprenant une séquence d'acides aminés ayant au moins 90 % d'identité de séquence avec SEQ ID N° : 2 lorsqu'elles sont alignées de manière optimale sur toute leur longueur à l'aide du programme GAP, et où la protéine AGXT I340M comprend une méthionine dans une position correspondant à la position 340 du SEQ ID N° : 2 ;
b) une molécule d'acide nucléique comprenant une séquence nucléotidique codant pour une protéine AGXT I340M telle qu'elle est définie en a); et
c) un virion d'un vecteur viral de thérapie génique comprenant une molécule d'acide nucléique telle qu'elle est définie en b).

4. Agent thérapeutique AGXT I340M destiné à être utilisé selon la revendication 1 ou la revendication 2 ou utilisation selon la revendication 3, dans lesquels la protéine AGXT I340M comprend des substitutions d'acides aminés sélectionnées dans le groupe constitué par :
a) une leucine dans une position correspondant à la position 11 du SEQ ID N°:2;
b) une arginine dans une position correspondant à la position 41 du SEQ ID N°: 2;
c) une isoleucine dans une position correspondant à la position 152 du SEQ ID N° : 2 ;
d) une arginine dans une position correspondant à la position 170 du SEQ ID N°: 2;
e) une thréonine dans une position correspondant à la position 244 du SEQ ID N°: 2;
f) une thréonine dans une position correspondant à la position 294 du SEQ ID N°: 2;
g) une isoleucine dans une position correspondant à la position 326 du SEQ ID N° : 2 ;
h) des combinaisons d'une ou de plusieurs des substitutions d'acides aminés b) à g); et
i) la combinaison des substitutions a) et f).

5. Agent thérapeutique AGXT I340M destiné à être utilisé selon la revendication 1 ou la revendication 2 ou utilisation selon la revendication 3, dans lesquels la protéine AGXT I340M est sélectionnée dans le groupe constitué par :
a) une protéine AGXT I340M comprenant la séquence d'acides aminés du SEQ ID N° : 2; et
b) une protéine AGXT I340M comprenant la séquence d'acides aminés du SEQ ID N° : 2 et comprenant une ou plusieurs substitutions d'acides aminés telles qu'elles sont définies dans la revendication 4.

6. Agent thérapeutique AGXT I340M destiné à être utilisé selon la revendication 1 ou la revendication 2 ou utilisation selon la revendication 3, dans lesquels l'agent thérapeutique AGXT I340M est une molécule d'acide nucléique comprenant une séquence nucléotidique codant pour une protéine AGXT I340M telle qu'elle est définie dans la revendication 4 ou 5.

7. Construction d'acide nucléique comprenant une séquence nucléotidique codant pour une protéine AGXT I340M telle qu'elle est définie dans l'une quelconque des revendications 1 à 6, dans laquelle la séquence nucléotidique est fonctionnellement liée à un promoteur de l'expression dans des cellules humaines et, facultativement, dans laquelle le promoteur n'est pas un promoteur issu d'un gène AGXT humain.

8. Construction d'acide nucléique selon la revendication 7, dans laquelle le promoteur est un promoteur spécifique du foie.

9. Construction d'acide nucléique selon la revendication 8, dans laquelle le promoteur spécifique du foie est sélectionné dans le groupe constitué par un promoteur de l'α1-antitrypsine (AAT), un promoteur de la globuline liant l'hormone thyroïdienne, un promoteur de l'albumine, un promoteur de la globuline liant la thyroxine (TBG), un promoteur hybride de la région de contrôle hépatique (HCR)-ApoCII, un promoteur hybride de l'HCR-hAAT, un promoteur de l'AAT combiné à l'élément d'amplification du gène de l'albumine de souris (Ealb) et un promoteur de l'apolipoprotéine E.

10. Construction d'acide nucléique selon la revendication 7, dans laquelle le promoteur a la séquence du SEQ ID N° : 3.

11. Construction d'acide nucléique selon l'une quelconque des revendications 7 à 10, dans laquelle la construction est un vecteur viral de thérapie génique, de préférence un vecteur parvoviral.

12. Virion de parvovirus comprenant une construction d'acide nucléique telle qu'elle est définie dans l'une quelconque des revendications 7 à 11.

13. Composition pharmaceutique comprenant un agent thérapeutique AGXT I340M destiné à être utilisé tel que défini dans l'une quelconque des revendications 1 à 6, une construction d'acide nucléique telle qu'elle est définie dans l'une quelconque des revendications 7 à 11 ou un virion de parvovirus tel qu'il est défini dans la revendication 12, et un véhicule pharmaceutiquement acceptable.

14. Construction d'acide nucléique telle qu'elle est définie dans l'une quelconque des revendications 7 à 11, virion de parvovirus tel qu'il est défini dans la revendication 12 ou composition pharmaceutique telle qu'elle est définie dans la revendication 13, à utiliser dans le traitement de l'hyperoxalurie primaire de type I.

15. Utilisation d'une construction d'acide nucléique telle qu'elle est définie dans l'une quelconque des revendications 7 à 11, d'un virion de parvovirus tel qu'il est défini dans la revendication 12 ou d'une composition pharmaceutique telle qu'elle est définie dans la revendication 13 dans la fabrication d'un médicament destiné à être utilisé dans le traitement de l'hyperoxalurie primaire de type I.
